(19) Europäisches Patentamt European Patent Office Office européen des brevets

(11) **EP 1 323 416 B1**

(12) **EUROPEAN PATENT SPECIFICATION**

(45) Date of publication and mention
of the grant of the patent:
**22.12.2010 Bulletin 2010/51**

(21) Application number: **01967818.4**

(22) Date of filing: **25.09.2001**

(51) Int Cl.:
*A61K 9/16* (2006.01)      *A61K 9/14* (2006.01)
*A61K 9/20* (2006.01)      *A61K 9/48* (2006.01)
*A61K 47/10* (2006.01)    *A61K 47/38* (2006.01)
*A61J 3/06* (2006.01)

(86) International application number:
**PCT/JP2001/008288**

(87) International publication number:
**WO 2002/024168 (28.03.2002 Gazette 2002/12)**

(54) **PROCESS FOR PRODUCING MEDICINAL SOLID DISPERSION**

VERFAHREN ZUR HERSTELLUNG EINER MEDIZINISCHEN FESTEN DISPERSION

PROCEDE DE PRODUCTION D'UNE DISPERSION SOLIDE MEDICINALE

(84) Designated Contracting States:
**DE FR GB IT**

(30) Priority: **25.09.2000 JP 2000289760**

(43) Date of publication of application:
**02.07.2003 Bulletin 2003/27**

(73) Proprietor: **Nippon Shinyaku Co., Ltd.
Kyoto-shi,
Kyoto 601-8550 (JP)**

(72) Inventors:
• **NAKANO, Tomio
Sakai-shi, Osaka 593-8302 (JP)**
• **TANAKA, Toshinori
Kyotanabe-shi, Kyoto 610-0343 (JP)**
• **IZUMI, Shogo
Kameoka-shi, Kyoto 621-0845 (JP)**

(74) Representative: **Vossius & Partner
Siebertstrasse 4
81675 München (DE)**

(56) References cited:
**EP-A- 0 580 860      EP-A- 0 852 140
WO-A-00/57854      FR-A- 2 702 968
US-A- 4 301 146**

• **LEUNER C ET AL: "Improving drug solubility for oral delivery using solid dispersions" EUROPEAN JOURNAL OF PHARMACEUTICS AND BIOPHARMACEUTICS, ELSEVIER SCIENCE PUBLISHERS B.V., AMSTERDAM, NL, vol. 50, no. 1, 3 July 2000 (2000-07-03), pages 47-60, XP004257179 ISSN: 0939-6411**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present invention relates to a process for producing a pharmaceutical solid dispersion which is useful as bulk substance of pharmaceutical preparations.

Background Art

The pharmaceutical solid dispersion is well-known among persons skilled in the art and is considered as such a state that a pharmaceutical ingredient in unimolecular form is dispersed in an inert carrier while being dissolved therein or in the solid state, as can be recognized from the fact that when such a pharmaceutical solid dispersion is analyzed using an X-ray diffraction apparatus, a crystalline peak of the pharmaceutical ingredient will not appear. Usually, the pharmaceutical solid dispersion is recognized as one of the useful devices for improving the solubility of pharmaceutical ingredients, in particular, water-insoluble pharmaceutical ingredients in living body, thereby improving the bioavailability.
As the inert carrier for the solid dispersion, water-soluble polymers such as methylcellulose, ethylcellulose, hydroxyethylcellulose, hydroxypropylcellulose, hydroxypropyl methylcellulose, carboxymetylcellulose, polyvinylpyrrolidone, polyvinyl alcohol, gum arabic, dextrin and gelatin are usually used. Among these hydroxypropyl methylcellulose is known as an excellent carrier for solid dispersion since it is superior from the view points of elution property and bioavailability of pharmaceutical ingredient, as well as it is stable toward moisture and the like (Chem. Pharm. Bull., 30, 4479 (1982); J.Pharm.Sci.Technol.,Jpn., 44, 31 (1984)).
On the other hand, as a technique for producing the solid dispersion, a solvent method, a fusion method, a solvent-fusion method and a mixing and grinding method are generally known. Moreover, recently, a method in which the solid dispersion is produced by executing kneading and extruding with the use of a kneading extruder comprising two screws, is also known, which is called a twin-screwcompounding extruder (See for example, PCT WO92/18106). The method of producing the solid dispersion by executing kneading and extruding processes using the twin-screw compounding extruder will not cause a problem of residual solvent as is often occurring in the solvent method, while providing many advantages such that it can be applied to many kinds of water-soluble polymer carriers and pharmaceutical ingredients, and a homogeneous and excellent solid dispersion can be continuously produced.
However, even if the solid dispersion is produced using hydroxypropyl methylcellulose which is superior in elution property of pharmaceutical ingredient and stability as a carrier while using the above-mentioned twin-screw compounding extruder, the obtainable solid dispersion is very hard, so that it is difficult to directly subject the solid dispersion to the subsequent preparation processes such as grinding and filling which follow the extruding. Granted that grinding is executed after extrusion, it will result in production of plenty of fibrous materials.
Further background art is represented by :

WO 00/57854 A, disclosing compositions obtained by twinscrew extrusion of the ingredients; an active principle and a surfactant are in a solid dispersion which furthermore can comprise HPMC or a sugar alcohol;

FR 2 702 968 A, disclosing a homogeneous mixture of active principle, hydrosoluble component (e.g. mannitol) as a cohesion agent, polymer (e.g. a cellulose derivative) as a binder/disintegrant for the preparation of particles by extrusion-lyophilization;

Disclosure of Invention

It is a primary object of the present invention to provide a method of producing a pharmaceutical solid dispersion using a twin-screw compounding extruder, the pharmaceutical solid dispersion including hydroxypropyl methylcellulose as a carrier which is superior in elution property of a pharmaceutical ingredient and in stability and the pharmaceutical solid dispersion being satisfactory for a subsequent pharmaceutical preparation.
As a result of earnest efforts, the inventors of the present invention found that the above-mentioned object can be achieved by using a sugar alcohol, as well as a pharmaceutical ingredient and hydroxypropyl methylcellulose which is a carrier, as essential components of a solid dispersion, to finally accomplish the present invention.
The present invention, therefore, provides a process for producing a pharmaceutical solid dispersion including hydroxypropyl methylcellulose as a carrier with the use of a twin-screw compounding extruder, **characterized in that** a sugar alcohol is added as one of processing materials and kneading and extruding processes are performed for the processing materials with the use of the twin-screw compounding extruder.
Sugar alcohols used in the present invention include, but not limited to, erythritol, mannitol, xylitol, sorbitol, inositol, maltitol, arabitol and dulcitol. Among these, erythritol, xylitol and mannitol are preferred, and erythritol is more preferred.

Pharmaceutical ingredients used in the present invention are not particularly limited, but water-insoluble pharmaceutical ingredients having a solubility at 25°C of 500 $\mu$g/mL or less with respect to the first liquid and the second liquid defined by the Japanese Pharmacopoeia, thirteenth edition, are suited, and water-insoluble pharmaceutical ingredients having a solubility at 25°C of 100 $\mu$g/mL or less with respect to the first fluid and the second fluid defined by the Japanese Pharmacopoeia, thirteenth edition, are preferred. In addition, water-insoluble pharmaceutical ingredients having such solubility and having a melting point or a decomposition temperature of not less than 50°C are more preferred. Concrete examples of such pharmaceutical ingredients are as follows.

1. Antipyresis, analgesia, antiphlogistine ingredients

Indometacin, aspirin, diclofenac sodium, ketoprofen, ibuprofen, mefenamic acid, dexamethasone, dexamethasone sodium sulfate, hydrocortisone, prednisolone, azulene, phenacetin, isopropylantipyrine, acetaminophen, benzydamine hydrochloride, phenylbutazone, flufenamic acid, sodium salicylate, choline salicylate, sasapyrine, clofezone, etodolac, and felbinac.

2. Antiulcer agents

Sulpiride, cetraxate hydrochloride, gefarnate, irsogladine maleate, cimetidine, ranitidine hydrochloride, famotidine, nizatidine, and roxatidine acetate hydrochloride.

3. Coronary vasodilators

Nifedipine, isosorbide dinitrate, diltiazem hydrochloride, trapidil, dipyridamole, dilazep hydrochloride, methyl 2,6-dimethyl-4-(2-nitrophenyl)-5-(2-oxo-1,3,2-dioxaphosphorinane-2-yl)-1,4-dihydropyridine-3-carboxylate, verapamil, nicardipine, nicardipine hydrochloride, and verapamil hydrochloride. 4. Peripheral vasodilator
Ifenprodil tartrate, cinepazide maleate, cyclandelate, cinnarizine, and pentoxifylline.

5. Antibiotics

Ampicillin, amoxycillin, cephalexin, erythromycin ethylsuccinate, bacampicillin hydrochloride, minocycline hydrochloride, chloramphenicol, tetracycline, erythromycin, griseofulvin, cefditoren pivoxil, azithromycin, and clarithromycin.

6. Synthetic antibacterial agents

Nalidixic acid, piromidic acid, pipemidic acid trihydrate, enoxacin, cinoxacin, ofloxacin, nortloxacin, ciprofloxacin hydrochloride, sulfamethoxazole trimethoprim, 6-fluoro-1-methyl-7-[4-(5-methyl-2-oxo-1,3-dioxolene-4-yl)methyl-1-piperazinyl]-4-oxo-4H [1,3] thiazeto [3,2-a] quinoline-3-carboxylic acid, and itraconazole.

7. Antispasmodics

Propantheline bromide, atropine sulfate, oxitropium bromide, timepidium bromide, scopolamine butylbromide, chlorination trospium, butropium bromide, N-methylscopolamine methyl sulfate, and methyloctatropin bromide.

8. Antitussive and antiasthmatic agents

Theophylline, aminophylline, methylephedrine hydrochloride, procaterol hydrochloride, trimetoquinol hydrochloride, codeine phosphate, sodium cromoglycate, tranilast, dextromethorphan hydrobromide, dimemorfan phosphate, clobutinol hydrochloride, fominoben hydrochloride, benproperine phosphate, tipepidine hibenzate, eprazinone hydrochloride, clofedanol hydrochloride, ephedrine hydrochloride, noscapine, carbetapentane citrate, oxeladin tannate, isoaminile citrate, pranlkast, and fluticasone propionate.

9. Bronchodilators

Diprophylline, salbutamol sulfate, clorprenaline hydrochloride, formoterol fumarate, orciprenaline sulfate, pirbuterol hydrochloride, hexoprenaline sulfate, bitolterol mesylate, clenbuterol hydrochloride, terbutaline sulfate, mabuterol hydrochloride, fenoterol hydrobromide, and methoxyphenamine hydrochloride.

10. Diuretics

Furosemide, acetazolamide, trichlormethiazide, methyclothiazide, hydrochlorothiazide, hydroflumethiazide, ethiazide, cyclopenthiazide, spironolactone, triamteren, chlorothiazide, piretanide, mefruside, ethacrynic acid, azosemide, and clofenamide.

11. Muscle relaxants

Chlorphenesin carbamate, tolperisone hydrochloride, eperisone hydrochloride, tizanidine hydrochloride, mephenesin, chlorzoxazone, phenprobamate, methocarbamol, chlormezanone, pridinol mesylate, aflogualone, baclofen, dantrolene sodium.

12. Cerebral metabolism betterment medicine

Meclofenoxate hydrochloride

13. Minor tranquilizers

Oxazolam, diazepam, clotiazepam, medazepam, temazepam, fludiazepam, meprobamate, nitrazepam, chlordiazepoxide, and quazepam.

14. Major tranquilizers

Sulpiride, clocapramine hydrochloride, zotepine, chlorpromazine, haloperidol, and risperidone.

15. β-blockers

Pindolol, propranolol hydrochloride, carteolol hydrochloride, metoprolol tartrate, labetalol hydrochloride, acebutolol hydrochloride, bufetolol hydrochloride, alprenolol hydrochloride, arotinolol hydrochloride, oxprenolol hydrochloride, nadolol, bucumolol hydrochloride, indenolol hydrochloride, timolol maleate, befunolol hydrochloride, bupranolol hydrochloride, and carvedilol.

16. Antiarrhythmics

Procainamide hydrochloride, disopyramide, ajmaline, quinidine sulfate, aprindine hydrochloride, propafenone hydrochloride, and mexiletine hydrochloride.

17. Gout remedies

Allopurinol, probenecid, colchicine, sulfinpyrazone, benzbromarone, and bucolome.

18. Anticoagulants

Ticlopidine hydrochloride, dicumarol, and warfarin potassium.

19. Antiepileptic agents

Phenytoin, sodium valproate, metharbital, and carbamazepine.

20. Antihistamine agents

Chlorpheniramine maleate, clemastine fumarate, mequitazine, alimemazine tartrate, cycloheptadine hydrochloride, and ebastin.

21. Antiemetic agents

Difenidol hydrochloride, metoclopramide, domperidone, betahistine mesylate, and trimebutine maleate.

22. Antihypertensive agents

Dimethylaminoethyl reserpinate hydrochloride, rescinnamine, methyldopa, prazosin hydrochloride, bunazosin hydrochloride, clonidine hydrochloride, budralazine, and urapidil.

23. Sympathomimetic agents

Dihydroergotamine mesylate, isoproterenol hydrochloride, and etilefrine hydrochloride.

24. Expectorants

Bromhexine hydrochloride, carbocysteine, ethylcysteine hydrochloride, and methylcysteine hydrochloride.

25. Oral diabetes mellitus therapeutic agents

Glibenclamide, tolbutamide, glymidine sodium, troglitazone, rosiglitazone, pioglitazone hydrochloride, and epalrestat.

26. Agents for circulatory organ

Ubidecarenone and ATP-2 Na.

27. Chalybeates

Ferrous sulfate and dry iron sulfate.

28. Vitamin preparations

Vitamin $B_1$, vitamin $B_2$, vitamin $B_6$, vitamin $B_{12}$, vitamin C, and folacin.

29. Pollakiuria therapeutic agents

Flavoxate hydrochloride, oxybutynin hydrochloride, terodiline hydrochloride, and 4-diethylamino-1,1-dimethyl-2-butynyl ($\pm$)-$\alpha$-cyclohexyl-$\alpha$-phenylglycolate hydrochloride monohydrate.

30. Angiotensin converting enzyme inhibitors

Enalapril maleate, alacepril, delapril hydrochloride, and candesartan cilexetil.

31. Nephritis therapeutic agents

(3$\beta$,4$\alpha$)-3,23-dihydroxy-N-(2-methoxyethyl)-18$\beta$-olean-12-ene-28-amide (hereinafter referred to as "Compound A").

32. Immunosuppressant

Tacrolimus.

33. Antineoplastic drugs

Paclitaxel, docetaxel and bicalutamide.

The present invention can be carried out by introducing each predetermined amount of a pharmaceutical ingredient, hydroxypropyl methylcellulose and a sugar alcohol in a mixture with an appropriate additive as desired, or simultaneously in separate conditions into a twin-screw compounding extruder according to a conventional method, executing processes such as kneading in the barrel of the twin-screw compounding extruder and extruding the processed object from the die. Some of processing materials may be introduced via an auxiliary feeding port on the extruder depending on the model of the extruder.

The twin-screw compounding extruder which may be used in the present invention is not particularly limited insofar as it comprises two screw shafts which allow temperature control of the barrel and the outlet die. Those having screw elements called kneading elements (also called kneading disc or paddle) along parts of the two screw shafts so as to oppose to each other are preferred, and a co-rotating intermeshing twin-screw compounding extruder having such screw elements is more preferred.

In the case of introducing a mixture of processing materials into the twin-screw compounding extruder, the mixing can be achieved either mechanically by means of a kneader mixer, a V-shape mixing machine, a double cone mixing machine, a cubic mixing machine, a ribbon-shape mixing machine and the like or manually in accordance with a conventional method.

Introduction of the processing materials into the barrel may be executed manually or by means of a material supplier generally provided in the extruder in use, however it can be executed without causing any restriction by an apparatus which are able to feed processing materials at a constant speed. Examples of such an apparatus include a screw feeder, a table feeder, a belt-conveyer type volumetric feeder, an electromagnetic feeder and the like.

Setting temperatures of the barrels and the die of the extruder are not particularly limited insofar as under that temperatures the processed object can be extruded and a solid dispersion is formed. Concretely, temperatures in the range of 130 to 250°C, preferably temperatures in the range of 170 to 200°C are suited. If the setting temperature is too high, the processed object may be discharged in a liquid form, which is undesirable from the view point of the posttreatment, and possibly leads decomposition of pharmaceutical ingredient. While on the other hand, if the setting temperature is too low, it is impossible to extrude the processed object, leading the fear that a solid dispersion cannot be produced.

The revolution of the screw (processing speed) can be appropriately set depending on the model or type of the extruder, as well as on the materials and the shape of the screw, and can be set within the tolerance of the extruder in use. The longer the overall barrel length of the extruder, the more the revolution can be increased. This is because performance of processes such as mixing and shearing becomes higher as the overall barrel length increases. Concretely, revolution of 20 rpm or more are suited, and revolution of 50 to 300 rpm are preferred.

The discharge pressure is suitably in the range of 10 to 200 $kg/cm^2$ and preferably in the range of 30 to 150 $kg/cm^2$.

The shape and combination of the screw elements which can be used in the present invention may be selected without particular limitation. However, it is preferred to use one or more set (s) of kneading elements (kneading disc, paddle) providing strong kneading action and shearing action.

The outlet die can be appropriately changed depending on the objective solid dispersion. Concrete examples of the outlet die include circular dies having various nozzle sizes for obtaining cylindrical processed objects, and flat dies for obtaining sheet-like processed objects.

The processed object that has been subjected to the processes such as kneading in the barrel of the twin-screw compounding extruder is then extruded continuously from a fine-sized nozzle of the die. This extruded object may be cut into a desired length by means of appropriate cutting machines such as a roller-type cracking machine, a cutter mill and a pin mill. This cut object may be directly or after dried, prepared into a granular pharmaceutical preparation. Also, by cutting the extrudate extruded from the fine-sized nozzle of the die into a desired length, for example, by means of a rotary cutter (for example, a rotary cutter for KEXN-30, available from Kurimoto, Ltd.) provided at the distal end of the die, it is possible to obtain a granular pharmaceutical preparation directly or after dried, without requiring any special granulating operation.

The above granular object may be rendered a capsule preparation by capsulation, or may be rendered a tablet preparation by compression molding.

It is also possible to encapsulate the granular object that has been subjected to a coating process or the like, or the granular object or its coated matter. This makes it possible to further improve the strength of the pharmaceutical preparation, as well as to improve the stability of the pharmaceutical ingredient.

It goes without saying that in the above preparation, the above-mentioned additives and polymer compounds may be appropriately added.

The weight ratio of hydroxypropyl methylcellulose and sugar alcohol (hydroxypropyl methylcellulose/sugar alcohol) is suitably in the range of 3 to 100, and preferably in the range of 5 to 20.

The blending ratio of the pharmaceutical ingredient is suitably in the range of 1 to 50% by weight in solid dispersion though it depends on the kind of the pharmaceutical ingredient, as well as on the blending ratio of hydroxypropyl methylcellulose and sugar alcohol, the kinds and amounts of other additives and the like. The weight ratio of hydroxypropyl methylcellulose and pharmaceutical ingredient (hydroxypropyl methylcellulose/pharmaceutical ingredient) is suitably in the range of 1 to 100, and preferably in the range of 3 to 10.

In addition to the above, pharmaceutically acceptable additives may be added as necessary to the solid dispersion produced by the process of the present invention. Examples of such additives include excipients (e.g., lactose, corn starch, crystalline cellulose, D-mannitol, calcium hydrogenphosphate), disintegrates (e.g., hydroxypropylcellulose of low substitution degree, carmellose, croscarmellose sodium, carmellose calcium), lubricants (e.g., magnesium stearate, calcium stearate, talc), colorants (e.g., iron sesquioxide, yellow iron sesquioxide, titanium oxide, tar dye), fragrant materials (e.g., 1-menthol, orange extract), surfactants (e.g., sucrose fatty acid ester, sodium lauryl sulfate, glycerine monostearate, polyoxyethylene hardened castor oil), and stabilizers (e.g., ascorbic acid, benzoic acid). The blending amount of such additives in solid dispersion is suitably 50% by weight or less.

Some quantity of pharmaceutically acceptable polymer compounds other than hydroxypropyl methylcellulose may be added as necessary as an additive for the purpose of adjusting release or the like rather than serving as a carrier. In principle, it is not necessary to contain other polymer compounds. Concrete examples of such polymers include cellulose derivatives such as methylcellulose, ethylcellulose, hydroxyethylcellulose, hydroxypropylcellulose, carboxymetylcellulose, carboxyethylcellulose, hydroxypropyl methylcellulose acetate succinate (AQOAT (registered trademark) L, AQOAT M, AQOAT H), hydroxypropyl methylcellulose phthalate (HP-55, HP-55S, HP-50), cellulose acetate phthalate, carboxymethyl ethylcellulose; acrylic acid derivatives such as polyacrylic acid, polymethacrylic acid and alkali salts of polymethacrylic acid, and copolymers of methacrylic acid (Eudragit (registered trademark) L30D55, Eudragit L100, Eudragit E100, Eudragit RL30D, Eudragit S100, Eudragit RL100, Eudragit RS100, Eudragit NE30D); polyvinylalcohol, polyvinylpyrrolidone, and macrogols. The blending amount of such polymer compounds in solid dispersion is suitably 20% by weight or less.

Best Mode for Carrying Out the Invention

The present invention will now be described in detail by way of examples, comparative examples and test examples. It goes without saying that the present invention is not limited to the examples as described below.

Example 1

To a Bohle container mixer (Model MC20, available from KOTOBUKI ENGINEERING & MANUFACTURING CO., LTD.), 200g of nifedipine, 1600g of hydroxypropyl methylcellulose (hereinafter abbreviated as "HPMC") 2910 and 200g of erythritol were introduced, and mixed for 20 minutes. The mixture was then subjected to kneading and extruding processes using a twin-screw compounding extruder (Model KEXN-30, available from KURIMOTO, LTD.) which includes kneading elements having a twist angle of 60° wherein the screw revolution is 50 rpm, the nozzle diameter of the die is 2 mm, and the temperature of the entire barrel and the die is set at 170°C, to thereby produce a pharmaceutical solid dispersion.

Example 2

A solid dispersion was produced in the same manner as Example 1 except that mannitol was used as the sugar alcohol and the temperature of the twin-screw compounding extruder was set at 170°C.

Example 3

A solid dispersion was produced in the same manner as Example 1 except that xylitol was used as the sugar alcohol and the temperature of the twin-screw compounding extruder was set at 170°C.

Example 4

A solid dispersion was produced in the same manner as Example 1 except that Compound A was used as the pharmaceutical ingredient and the temperature of the twin-screw compounding extruder was set at 190°C.

Example 5

A solid dispersion was produced in the same manner as Example 1 except that griseofulvin was used as the pharmaceutical ingredient and the temperature of the twin-screw compounding extruder was set at 190°C.

Example 6

A solid dispersion was produced in the same manner as Example 1 except that phenytoin was used as the pharmaceutical ingredient and the temperature of the twin-screw compounding extruder was set at 190°C.

Comparative example 1

To a Bohle container mixer, 200g of nifedipine and 1600g of HPMC 2910 were introduced, and mixed for 20 minutes. The mixture was then subjected to kneading and extruding processes using the twin-screw compounding extruder which includes kneading elements having a twist angle of 60° wherein the screw revolution is 50 rpm, the nozzle diameter of the die is 2 mm, and the temperature of the entire barrel and the die is set at 210°C, to thereby produce a pharmaceutical solid dispersion.

Comparative example 2

A solid dispersion was produced in the same manner as Comparative example 1 except that the blending amount of HPMC 2910 was 1800 g.

Comparative example 3

A solid dispersion was produced in the same manner as Comparative example 1 except that Compound A was used as the pharmaceutical ingredient and the temperature of the twin-screw compounding extruder was set at 200°C.

Comparative example 4

A solid dispersion was produced in the same manner as Comparative example 1 except that the blending amount of HPMC 2910 was 1800 g, Compound A was used as the pharmaceutical ingredient, and the temperature of the twin-screw com pounding extruder was set at 200°C.

Comparative example 5

A solid dispersion was produced in the same manner as Comparative example 1 using griseofulvin as the pharmaceutical ingredient.

Comparative example 6

A solid dispersion was produced in the same manner as Comparative example 1 except that griseofulvin was used as the pharmaceutical ingredient and the blending amount of HPMC 2910 was 1800 g.

Comparative example 7

A solid dispersion was produced in the same manner as Comparative example 1 except that phenytoin was used as the pharmaceutical ingredient and the temperature of the twin-screw compounding extruder was set at 200°C.

Comparative example 8

A solid dispersion was produced in the same manner as Comparative example 1 except that the blending amount of HPMC 2910 was 1800 g, phenytoin was used as the pharmaceutical ingredient, and the temperature of the twin-screw compounding extruder was set at 200°C.

Test example 1 (Pulverizing experiment)

The solid dispersions (extrudates) obtained by Example 1 and Comparative example 1 were pulverized using a sample mill (Model AP-S, available from HOSOKAWAMICRON CORPORATION.) A punch screen having a pore size of 1 mm was used.
It was revealed that the solid dispersion according to Comparative example 1 in which a sugar alcohol is not blended was difficult to be pulverized and plenty of fibrous materials existed. To the contrary, the solid dispersion according to Example 1 in which a sugar alcohol is blended was easily pulverized, and little fibrous materials existed.

Test example 2 (Measurement of tensile strength)

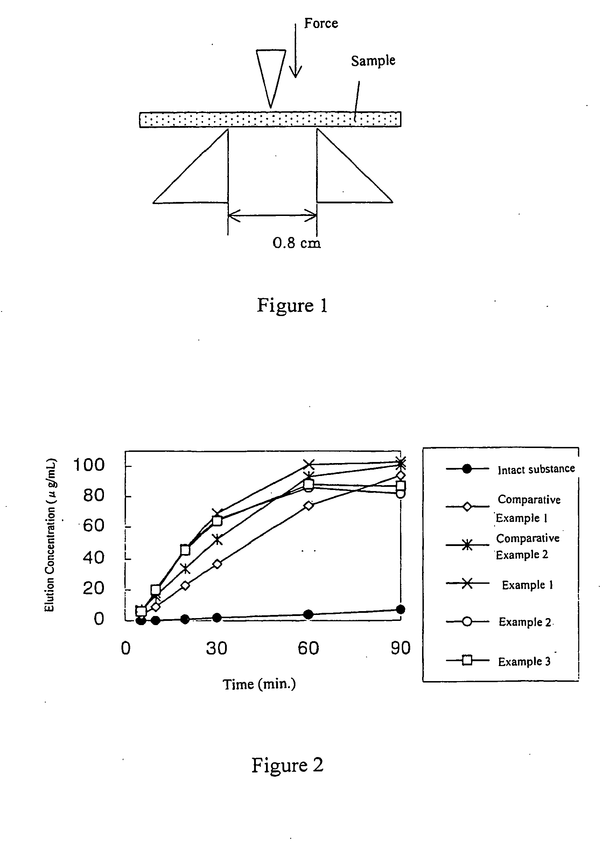

Strength was measured for the solid dispersions (extrudates) obtained by Example 1, Comparative example 1 and Comparative example 2 under the condition of Fig. 1 with the use of an autograph (AG-5000G, available from SHIMADZU CORPORATION; compression speed 1mm/min.), and tensile strength ($\sigma$ max) was calculated from the following formula:

$$\sigma \max \ (N/cm^2) = 4QX/\pi r^3$$

Q: maximum load at the time of sample breakage (N)
X: distance between supporting point and loading point (0.4 cm)
r: radius of sample (1 mm)

The results are shown in Table 1 below.

Table 1

| Nif.: HPMC: erythritol | 1:8:0 | 1:9:0 | 1:8:1 |
|---|---|---|---|
| Strength (N) $\pm$ S.D. | 61.7 $\pm$ 1.4 | 59.8 $\pm$ 1.0 | 31.9 $\pm$ 4.7 |
| Tensile strength (N/cm$^2$) | 31.5 | 30.5 | 16.3 |
| Nif.: nifedipine | | | |

As is apparent from Table 1, it was found that the solid dispersion containing a sugar alcohol has a lower tensile strength lower than the solid dispersion not including a sugar alcohol, and hence the solid dispersion containing a sugar alchol is pulverized more easily.

Test example 3 (Elution Test)

With respect to a solid dispersion sample (16 to 32 mesh (500 to 1,000 $\mu$m) sample) in an amount corresponding to 50 mg of a pharmaceutical ingredient or an intact pharmaceutical ingredient of 50 mg, elution amount (dissolution amount) of the pharmaceutical ingredient from the solid dispersion or dissolution concentration of the intact pharmaceutical ingredient was determined in accordance with the Method 2 of Dissolution Test (paddle method) defined in the Japanese Pharmacopoeia, 13th edition. The test was carried out using 500 mL of purified water as a test solution and puddle rotation of 100 rpm. After starting the test, 2.5 mL of eluate was sampled at predetermined intervals, and filtered by a membrane filter. 1 mL of internal standard solution was added to each 1 mL of filtrate and the elution concentration or the dissolution concentration was determined by the HPLC method.

The results are shown in Figures 2 to 5.

As is apparent from each figure, the elution amounts (the dissolution amounts) of pharmaceutical ingredients from the solid dispersions containing a sugar alcohol were not inferior to those of the solid dispersions not containing a sugar alcohol.

Effect of the Invention

According to the present invention,

(1) in producing a pharmaceutical solid dispersion including HPMC as a carrier which is superior in elution property and stability of pharmaceutical ingredients while using a twin-screw compounding extruder, it is possible to set the process temperature lower than that of the case where a sugar alcohol is not blended,
(2) the extruded solid dispersion can be easily crushed, and
(3) the pulverized object includes little fibrous materials. Since little fibrous materials are included in the pulverized object, it is possible to reduce segregation in preparation processes such as mixing and screening, while it is possible to realize uniform filling (with small weight variation) in a tableting or encapsulation process. Furthermore, it is possible to reduce the troubles by entering the pulverized object into a space existing in equipment used for preparation.

Brief Explanation of Drawings

Figure 1 is a schematic view of a measurement method for determining tensile strength.
Figure 2 shows results of elution test of nifedipine from solid dispersion. The vertical axis represents elution concentration ($\mu$g/mL), and the horizontal axis represents time (min.). The curve denoted by -•- shows transition in elution concentration of intact nifedipine, the curve denoted by -X- shows transition in elution concentration of nifedipine from solid dispersion according to Example 1, the curve denoted by -○- shows transition in elution concentration of nifedipine from solid dispersion according to Example 2, the curve denoted by -□- shows transition in elution concentration of nifedipine from solid dispersion according to Example 3, the curve denoted by -◊- shows transition in elution concentration of nifedipine from solid dispersion according to Comparative example 1, and the curve denoted by -*- shows transition in elution concentration of nifedipine from solid dispersion according to Comparative example 2.

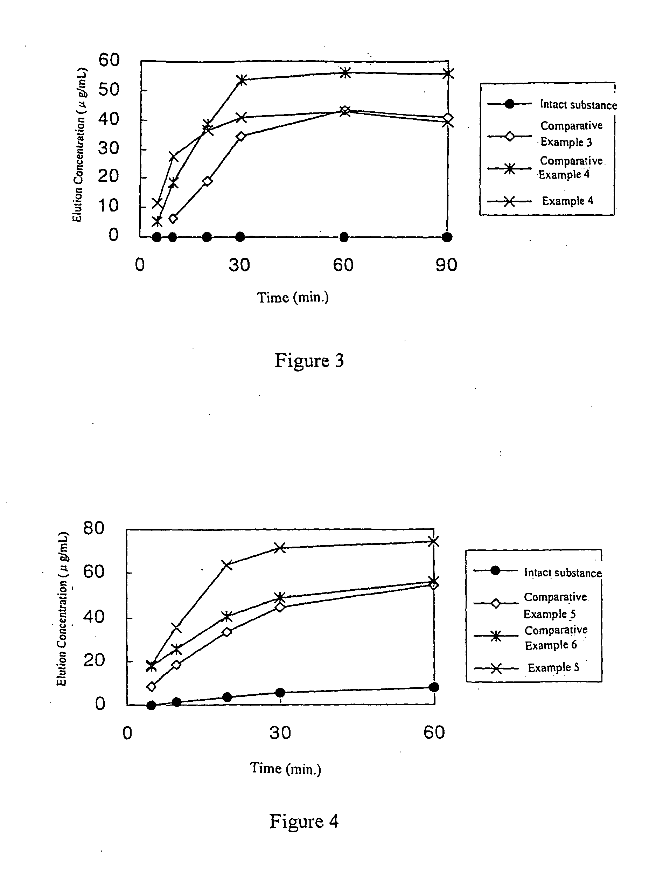

Figure 3 shows results of elution test of Compound A from solid dispersion. The vertical axis represents elution concentration ($\mu$g/mL), and the horizontal axis represents time (min.). The curve denoted by -•- shows transition in elution concentration of intact Compound A, the curve denoted by -X- shows transition in elution concentration of Compound A from solid dispersion according to Example 4, the curve denoted by -◊-shows transition in elution concentration of Compound A from solid dispersion according to Comparative example 3, and the curve denoted by -*- shows transition in elution concentration of Compound A from solid dispersion according to Comparative example 4.

Figure 4 shows results of elution test of griseofulvin from solid dispersion. The vertical axis represents elution concentration ($\mu$g/mL), and the horizontal axis represents time (min.). The curve denoted by -•- shows transition in elution concentration of intact griseofulvin, the curve denoted by -X- shows transition in elution concentration of griseofulvin from solid dispersion according to Example 5, the curve denoted by -◊-shows transition in elution concentration of griseofulvin from solid dispersion according to Comparative example 5, and the curve denoted by -*- shows transition in elution concentration of griseofulvin from solid dispersion according to Comparative example 6.

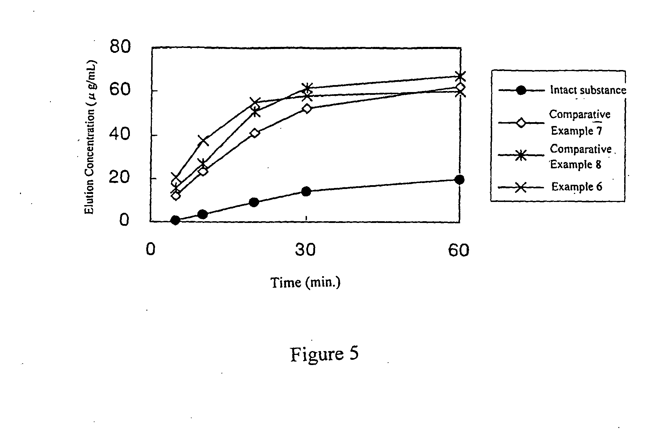

Figure 5 shows results of elution test of phenytoin from solid dispersion. The vertical axis represents elution concentration ($\mu$g/mL), and the horizontal axis represents time (min.). The curve denoted by -•- shows transition in elution concentration of intact phenytoin, the curve denoted by -X- shows transition in elution concentration of phenytoin from solid dispersion according to Example 6, the curve denoted by -◊- shows transition in elution concentration of phenytoin from solid dispersion according to Comparative example 7, and the curve denoted by -*- shows transition in elution concentration of phenytoin from solid dispersion according to Comparative example 8.

## Claims

1. A process for producing a pharmaceutical solid dispersion including hydroxypropyl methylcellulose as a carrier with the use of a twin-screw compounding extruder, **characterized in that** a sugar alcohol is added as one of processing materials and kneading and extruding processes are performed for the processing materials with the use of the twin-screw compounding extruder, followed by pulverizing or cutting the extrudate.

2. The process for producing a pharmaceutical solid dispersion according to claim 1, wherein the sugar alcohol is at least one selected from the group consisting of erythritol, mannitol, xylitol, sorbitol, inositol, maltitol, arabitol and dulcitol.

3. The process for producing a pharmaceutical solid dispersion according to claim 1 or 2, wherein the pharmaceutical ingredient is a water-insoluble pharmaceutical ingredient having a solubility at 25˚C of 500 $\mu$g/mL or less with respect to the first fluid and the second fluid defined by the Japanese Pharmacopoeia, thirteenth edition.

4. The process for producing a pharmaceutical solid dispersion according to claim 3, wherein the pharmaceutical ingredient is a water-insoluble pharmaceutical ingredient having a melting point or a decomposition temperature of not less than 50˚C.

5. The process for producing a pharmaceutical solid dispersion according to claim 1, wherein a weight ratio between hydroxypropyl methylcellulose and pharmaceutical ingredient (hydroxypropyl methylcellulose/pharmaceutical ingredient) is in the range of 1 to 100.

6. The process for producing a pharmaceutical solid dispersion according to claim 1, wherein a weight ratio between hydroxypropyl methylcellulose and sugar alcohol (hydroxypropyl methylcellulose/sugar alcohol) is in the range of 3 to 100.

7. The process for producing a pharmaceutical solid dispersion according to claim 1, wherein the twin-screw compounding extruder is a co-rotating intermeshing twin-screw compounding extruder.

8. The process for producing a pharmaceutical solid dispersion according to claim 1 or 7, wherein the setting temperatures of each barrel and die of the twin-screw compounding extruder which are the same or different, fall within the range of 130°C to 250°C.

**Patentansprüche**

1. Verfahren zur Herstellung einer pharmazeutischen festen Dispersion, welche Hydroxypropylmethylcellulose als einen Träger beinhaltet, unter Verwendung eines Doppelschnecken-Compoundierextruders, **dadurch gekennzeichnet, dass** ein Zuckeralkohol als eines der Verarbeitungsmaterialien hinzugefügt wird und Knet- und Extrudiervorgänge für die Verarbeitungsmaterialien durchgeführt werden unter Verwendung des Doppelschnecken-Compoundierextruders, gefolgt vom Pulverisieren oder Zerschneiden des Extrudats.

2. Verfahren zur Herstellung einer pharmazeutischen festen Dispersion nach Anspruch 1, wobei der Zuckeralkohol mindestens einer ist ausgewählt aus der Gruppe bestehend aus Erythrit, Mannit, Xylit, Sorbit, Inosit, Maltit, Arabit und Dulcit.

3. Verfahren zur Herstellung einer pharmazeutischen festen Dispersion nach Anspruch 1 oder 2, wobei der pharmazeutische Bestandteil ein wasserunlöslicher pharmazeutischer Bestandteil ist, der bei 25 °C eine Löslichkeit von 500 μg/ml oder weniger bezüglich der ersten Flüssigkeit und der zweiten Flüssigkeit hat, wie nach der japanischen Pharmacopoeia, dreizehnte Ausgabe, definiert.

4. Verfahren zur Herstellung einer pharmazeutischen festen Dispersion nach Anspruch 3, wobei der pharmazeutische Bestandteil ein wasserunlöslicher pharmazeutischer Bestandteil ist, der einen Schmelzpunkt oder eine Zersetzungstemperatur von nicht weniger als 50 °C hat.

5. Verfahren zur Herstellung einer pharmazeutischen festen Dispersion nach Anspruch 1, wobei das Gewichtsverhältnis zwischen Hydroxypropylmethylcellulose und pharmazeutischem Bestandteil (Hydroxypropylmethylcellulose/pharmazeutischer Bestandteil) im Bereich von 1 bis 100 liegt.

6. Verfahren zur Herstellung einer pharmazeutischen festen Dispersion nach Anspruch 1, wobei das Gewichtsverhältnis zwischen Hydroxypropylmethylcellulose und Zuckeralkohol (Hydroxypropylmethylcellulose/Zuckeralkohol) im Bereich von 3 bis 100 liegt.

7. Verfahren zur Herstellung einer pharmazeutischen festen Dispersion nach Anspruch 1, wobei der Doppelschnecken-Compoundierextruder ein gleichlaufender ineinandergreifender Doppelschnecken-Compoundierextruder ist.

8. Verfahren zur Herstellung einer pharmazeutischen festen Dispersion nach Anspruch 1 oder 7, wobei die für jeden Zylinder und jede Düse des Doppelschnecken-Compoundierextruders eingestellten Temperaturen, die jeweils gleich oder verschieden sind, in den Bereich zwischen 130 °C und 250 °C fallen.

**Revendications**

1. Procédé de production d'une dispersion solide pharmaceutique comprenant de l'hydroxypropyl méthylcellulose en tant que support avec l'utilisation d'une extrudeuse-mélangeuse à double vis, **caractérisé en ce qu'**un polyol est ajouté en tant qu'un des matériaux de transformation et **en ce que** les procédés de pétrissage et d'extrusion sont réalisés sur les matériaux de transformation avec l'utilisation de l'extrudeuse-mélangeuse à double vis, suivi par la pulvérisation ou le découpage de l'extrudat.

2. Procédé de production d'une dispersion solide pharmaceutique selon la revendication 1, où le polyol est au moins un polyol choisi parmi le groupe consistant en érythritol, mannitol, xylitol, sorbitol, inositol, maltitol, arabitol et dulcitol.

3. Procédé de production d'une dispersion solide pharmaceutique selon les revendications 1 ou 2, où l'ingrédient pharmaceutique est un ingrédient pharmaceutique insoluble dans l'eau ayant une solubilité à 25°C de 500 μg/ml ou moins par rapport au premier fluide et au deuxième fluide définie par *Japanese Pharmacopoeia,* 13ᵉ édition.

4. Procédé de production d'une dispersion solide pharmaceutique selon la revendication 3, où l'ingrédient pharmaceutique est un ingrédient pharmaceutique insoluble dans l'eau ayant un point de fusion ou une température de décomposition non inférieure à 50˚ C.

5. Procédé de production d'une dispersion solide pharmaceutique selon la revendication 1, où un rapport en poids entre l'hydroxypropyl méthylcellulose et l'ingrédient pharmaceutique (hydroxypropyl méthylcellulose/ingrédient pharmaceutique) est compris dans le domaine de 1 à 100.

6. Procédé de production d'une dispersion solide pharmaceutique selon la revendication 1, où un rapport en poids entre l'hydroxypropyl méthylcellulose et le polyol (hydroxypropyl méthylcellulose/polyol) est compris dans le domaine de 3 à 100.

7. Procédé de production d'une dispersion solide pharmaceutique selon la revendication 1, où l'extrudeuse-mélangeuse à double vis est une extrudeuse-mélangeuse à double vis engrenantes à co-rotation.

8. Procédé de production d'une dispersion solide pharmaceutique selon les revendications 1 ou 7, où les températures de durcissement de chaque cylindre et filière de l'extrudeuse-mélangeuse à double vis qui sont identiques ou différentes, sont comprises dans le domaine de 130˚C à 250˚C.

Figure 1

Figure 2

Figure 3

Figure 4

Figure 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9218106 A
- WO 0057854 A

- FR 2702968 A

**Non-patent literature cited in the description**

- *Chem. Pharm. Bull.,* 1982, vol. 30, 4479

- *J.Pharm.Sci.Technol.,Jpn.,* 1984, vol. 44, 31